# EUROPEAN PATENT APPLICATION

(11) **EP 4 715 385 A1**
(43) Date of publication of application: **25.03.2026**
(21) Application number: 24811065.2
(22) Date of filing: 17.05.2024
(51) Int. Cl.: G01N 33/68, G01N 33/70

(54) **METHOD FOR TESTING TERTIARY LYMPHOID TISSUE, AND KIT FOR TESTING TERTIARY LYMPHOID TISSUE**

(30) Priority: 19.05.2023 JP 2023083082
(71) Applicant: Kyoto University, Kyoto-shi, Kyoto 606-8501 (JP)
(72) Inventor: YANAGITA, Motoko, Kyoto-shi, Kyoto 606-8501 (JP); SUGIURA, Yuki, Kyoto-shi, Kyoto 606-8501 (JP); ARAI, Hiroyuki, Kyoto-shi, Kyoto 606-8501 (JP)
(74) Representative: J A Kemp LLP
(86) International application number: PCT/JP2024/018364
(87) International publication number: WO 2024/242058

(57) **Abstract**

One purpose of the present disclosure is to provide a technique for testing the presence or absence of a tertiary lymphoid tissue in a subject without conducting biopsy. The method for testing the presence or absence of a tertiary lymphoid tissue in a subject comprises a step for measuring the concentration of glutathione in a bodily fluid sample collected from the subject.

## Description

### Technical Field

The present disclosure relates to a method for testing the presence or absence of a tertiary lymphoid tissue (TLT) in a subject. The present disclosure also relates to a kit for testing the presence or absence of a TLT in a subject.

### Background Art

The TLT is an inducible ectopic lymphoid tissue in which a tissue similar to a lymph node is formed in a non-lymphoid tissue (NPLs 1 to 3). The TLT, which is heavily infiltrated by lymphocytes, amplifies an immune reaction as a starting point of acquired immune response via interaction and proliferation of T cells and B cells. The TLT is known to be formed in a lesion such as a disease accompanied by chronic inflammation, an autoimmune disease, an infectious disease, or cancer (NPLs 1 to 3) and to affect the severity and prognosis of the diseases.

For example, in kidney diseases such as pyelonephritis, IgA nephropathy, and lupus nephritis, it is known that protraction and repair failure of inflammation occur when a TLT is induced (NPL 4). In addition, in recent years, the present inventors have revealed that the degree of maturation of a TLT can be used as a biomarker reflecting the degree of tissue injury of the kidney (NPL 5). Specifically, as a result of tissue analysis of human pyelonephritis, it has been revealed that TLTs at various stages of maturation occur simultaneously in the kidney, and TLTs having a higher degree of maturation are observed in a part where tissue damage is severe, whereas induction of TLTs is inconspicuous in a part where damage is mild, and the proportion of immature TLTs is also high in the formed TLTs. In analysis of the elderly, it has been also confirmed that the number of TLTs is significantly larger in the cases of complicated chronic kidney disease than in the cases of non-complicated chronic kidney disease, and the proportion of TLTs having a higher degree of maturation is high. Furthermore, it has been confirmed that a TLT is induced to the same anatomical site through the same stage of maturation, regardless of the type of underlying disease. In addition, it has been reported that when there is a TLT having a high degree of maturation in the kidney after transplantation, deterioration in renal function after 5 years is likely to be observed (NPL 6) and considered that the TLT can be a predictive marker of future renal function.

As described above, it is known that the TLT may affect the severity, prognosis, and responsiveness to treatment of the disease, and confirmation of the presence or absence of formation and the degree of maturation of the TLT is useful for determining a policy regarding therapeutic intervention and determining the effect.

On the other hand, in order to confirm the presence or absence of formation of the TLT, it is necessary to collect tissue of a lesion site and perform biopsy. However, the collection of the tissue of the lesion site requires the posture to keep constant for a predetermined time, there is also a risk of bleeding and the like, this is, a disadvantage that the burden on the patient is large. In addition, there is a high risk of tissue collection in patients under treatment with antiplatelet drugs and elderly people, making particularly difficult to confirm the presence or absence of formation of a TLT in the patients.

### Citation List

### Non Patent Literature

NPL1: Sato Y. et al., Immunology of the ageing kidney. Nat Rev Nephrol. 2019;15(10):625-40.
NPL2: Antonioli L. et al. Ectopic Lymphoid Organs and Immune-Mediated Diseases: Molecular Basis for Pharmacological Approaches. Trends Mol Med. 2020;26(11):1021-1033.
NPL3: Sautes-Fridman C. et al. Tertiary lymphoid structures in the era of cancer immunotherapy. Nat Rev Cancer. 2019;19(6):307-25.
NPL4: Sato Y. et al. Heterogeneous fibroblasts underlie age-dependent tertiary lymphoid tissues in the kidney. JCI Insight. 2016;1(11):e87680.
NPL5: Sato Y. et al. Developmental stages of tertiary lymphoid tissue reflect local injury and inflammation in mouse and human kidneys. Kidney Int. 2020;98(2):448-463.
NPL6: Lee H. et al., HYPERLINK "https://pubmed.ncbi.nlm.nih.gov/34725107/"Advanced Tertiary Lymphoid Tissues in Protocol Biopsies are Associated with Progressive Graft Dysfunction in Kidney Transplant Recipients. J Am Soc Nephrol. 2022;33(1):186-200. doi: 10.1681/ASN.2021050715.

### Summary of Invention

### Technical Problem

One object of the present disclosure is to provide a technique for testing the presence or absence of a TLT in a subject without biopsy.

### Solution to Problem

The present inventors have extensively conducted studies for achieving the object, and as a result, found that in a mouse in which a TLT is formed in the kidney, glutathione is accumulated in the TLT, and the concentration of glutathione in a urine sample increases. Furthermore, the present inventors have analyzed the concentration of glutathione in a urine sample by grouping patients with IgA nephropathy according to the presence or absence of a TLT and found that the concentration of glutathione in the urine sample significantly increases in patients having a TLT as compared with patients without a TLT. The present disclosure has been completed by further conducting studies based on these findings.

That is, the present disclosure provides inventions of the following aspects.
Item 1. A testing method for determining presence or absence of a TLT in a subject, the testing method including a step of measuring a glutathione concentration in a body fluid sample collected from the subject.
Item 2. The testing method according to item 1, wherein the body fluid sample is a urine sample.
Item 3. The testing method according to item 1 or 2, wherein the glutathione concentration is a reduced glutathione concentration.
Item 4. The testing method according to item 2, further including a step of measuring a creatinine concentration in the urine sample.
Item 5. The testing method according to any one of items 1 to 4, wherein the subject is a kidney disease patient, a person in need of testing presence or absence of kidney disease infection, or a person who has undergone kidney transplantation, the testing method being performed for testing presence or absence of a TLT in a kidney.
Item 6. A test kit for testing presence or absence of a TLT in vivo, the test kit including a reagent for measuring a glutathione concentration.
Item 7. Use of a reagent for measuring a glutathione concentration in manufacture of a test kit for testing presence or absence of a TLT in a body.

### Advantageous Effects of Invention

According to an embodiment of the testing method of the present disclosure, the use of the glutathione concentration in a body fluid sample such as urine as a biomarker of a TLT making it possible to test the presence or absence of a TLT in a subject without biopsy. For example, by predicting the presence or absence of a TLT in the kidney of a kidney disease patient or a person who has undergone kidney transplantation by the testing method of the present disclosure, the degree of renal disorder, the function of the transplanted kidney, and the like can be estimated, enabling early treatment intervention for a patient having a high risk of deterioration of the function of the transplanted kidney.

### Brief Description of Drawings

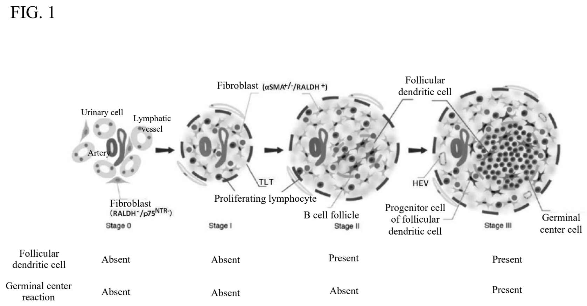

[Fig. 1] Fig. 1 shows a schematic structural diagram for each degree of maturation of a TLT formed in the kidney.

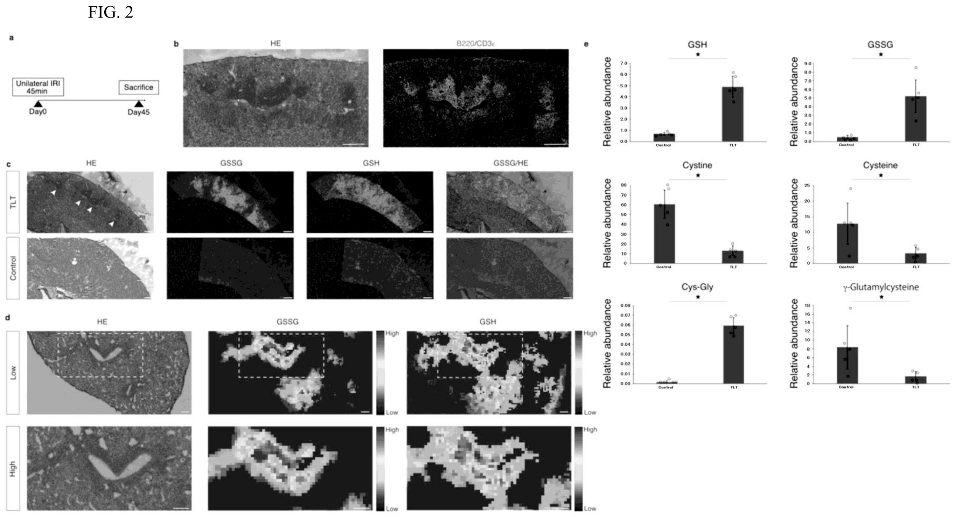

[Fig. 2] Fig. 2a shows an experimental scheme when the amount of glutathione in kidney tissue and urine is measured for a kidney TLT model produced by induction of ischemia reperfusion injury (IRI). Fig. 2b shows an image of renal tissues of IRI-induced mice stained with HE (left) and immunostained with an anti-CD3e antibody and an anti-B 220 antibody (right). Fig. 2c shows an HE stained image (left end), a mass imaging (MSI) image of oxidized glutathione (GSSG) (second from the left), an MSI image of reduced glutathione (GSH) (second from the right), and an image in which the HE stained image and the MSI image of GSSG are merged (right end), of the renal tissues of a mouse having induced IRI and a control mouse, and in the HE stained image of Fig. 2c, formation of a TLT is observed in the arrow portion. Fig. 2d is an HE stained image (left) obtained by enlarging a region where TLTp is formed in the renal tissue of a mouse in which IRI is induced, an MSI image (middle) of GSSG, and an MSI image (right) of GSH, and in Fig. 2d, an image obtained by further enlarging a region surrounded by a dotted line of the image (Low) illustrated in the upper part is the image (High) illustrated in the lower part. Fig. 2e shows the results of measuring the relative amounts of GSH, GSSG, cystine, cysteine, dipeptide Cys-Gly, and γ-glutamylcysteine extracted from the renal tissues of IRI-induced mice and control mice, and in Fig. 2e, * indicates p < 0.05.

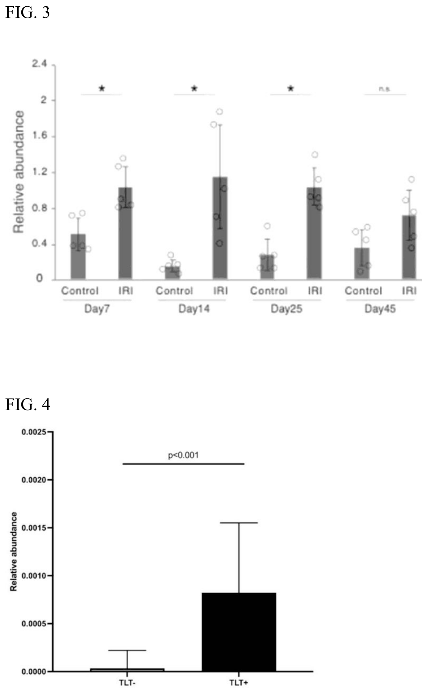

[Fig. 3] Fig. 3 shows the results of determining the relative values of the urinary reduced glutathione concentration (relative values with respect to urinary creatinine concentration of 1) of IRI-induced mice and control mice. In Fig. 3, * indicates p < 0.05, and n. s. indicates no significant difference.
[Fig. 4] Fig. 4 shows the results of determining relative values of the urinary reduced glutathione concentration (relative values with respect to urinary creatinine concentration of 1) by dividing IgA nephropathy patients into a group with a TLT (TLT+) and a group without a TLT (TLT-).

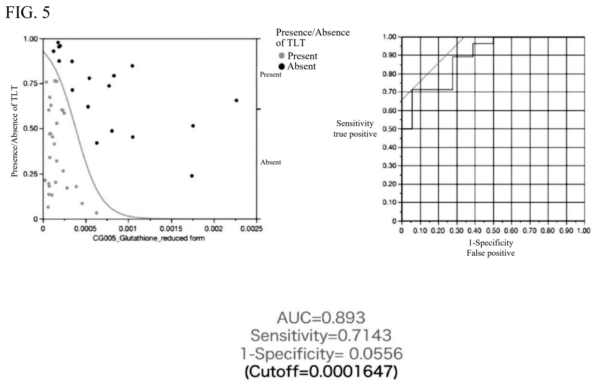

[Fig. 5] Fig. 5 is a diagram showing the results of performing logistic regression analysis and ROC analysis on IgA nephropathy patients, using a relative value of urinary reduced glutathione concentration (relative value with respect to urinary creatinine concentration of 1) as an explanatory variable, with patients in the group with a TLT being positive (1) and patients in the group without a TLT being negative (0).

### Description of Embodiments

### 1. Terms

Unless specifically defined otherwise, the terms used herein have the meanings as commonly understood by those skilled in the art of medicine, pharmacy, molecular biology, microbiology, organic chemistry, and the like. When a term defined herein does not have the same meaning as commonly understood, the description herein prevails.

In the present disclosure, the term "tertiary lymphoid tissue (TLT)" refers to a tissue similar to a lymph node and an inducible ectopic lymphoid tissue that is composed of an aggregate of lymphocytes and formed in a non-lymphoid tissue. In the present disclosure, the expression "degree of maturation of the tertiary lymphoid tissue (TLT)" refers to the degree of structural maturation of the TLT. In the present disclosure, the degree of maturation of the tertiary lymphoid tissue (TLT) is classified into stages I to III as follows according to the classification described in NPL 5. For reference, Fig. 1 shows a schematic structural diagram for each degree of maturation of a TLT formed in the kidney. Stage 0 shown in Fig. 1 is a precursor of the TLT in which lymphocytes are not organized, and does not correspond to the TLT in the present disclosure.
Stage I: TLT without follicular dendritic cells (FDCs) and germinal centers
Stage II: TLT with FDCs but without germinal centers
Stage III: TLT with FDCs and germinal centers

In the present disclosure, the term "glutathione" refers to a concept including both reduced glutathione and oxidized glutathione. The reduced glutathione is a tripeptide in which glutamic acid, cysteine, and glycine are peptide-linked in this order. However, in the reduced glutathione, glutamic acid and cysteine have an amide bond between a γ-carboxyl group of a side chain of glutamic acid and an amino group of cysteine. The oxidized glutathione is a molecule in which two reduced glutathione molecules are linked by a disulfide bond.

### 2. Testing Method for TLT

The testing method of the present disclosure is a testing method for determining the presence or absence of a TLT in a subject, including the step of measuring a glutathione concentration in a body fluid sample collected from the subject. Hereinafter, the testing method of the present disclosure will be described in detail.

### [Subject]

In the testing method of the present disclosure, the term "subject" refers to a human or non-human animal to be tested for determining the presence or absence of formation of a TLT in vivo. Examples of the non-human animal include non-human mammals such as primates, rats, mice, Mongolian gerbils, guinea pigs, hamsters, ferrets, rabbits, cows, horses, pigs, goats, dogs, and cats. Preferred examples of the subject include humans.

The subject in the testing method of the present disclosure may be any subject as long as it is required to test the presence or absence of formation of a TLT in vivo, and preferred examples thereof include a person suffering from a disease in which formation of a TLT occurs at a lesion, a person in need of testing the presence or absence of the disease infection, and a person who has undergone organ transplantation for treatment of the disease. Examples of the disease in which the formation of the TLT occurs in the lesion include a disease accompanied by chronic inflammation, chronic kidney disease, obesity, asthma, allergic lung disease, arteriosclerosis, autoimmune disease, infectious disease, and cancer.

Specific examples of the subject in the testing method of the present disclosure include a kidney disease patient and a person in need of testing the presence or absence of kidney disease infection. The type of kidney disease is not particularly limited, and examples thereof include IgA nephropathy, lupus nephritis, ANCA-associated vasculitis, diabetic nephropathy, nephrosclerosis, pyelonephritis, and chronic renal failure. Among these kidney diseases, IgA nephropathy is preferable. In kidney disease, as the renal dysfunction progresses, the TLT tends to be formed in the lesion and the degree of maturation thereof tends to increase, and therefore, in the testing method of the present disclosure, when a subject is a person suffering from kidney disease or a person in need of testing the presence or absence of kidney disease infection, the presence or absence of kidney disease infection can be estimated in addition to the presence or absence of TLT.

In addition, another specific example of the subject in the testing method of the present disclosure includes a person who needs prediction of future renal function. Examples of the person whose future renal function needs to be predicted include a kidney disease patient and a person who has undergone kidney transplantation. For example, in a case where a subject is a person who has undergone kidney transplantation, the prognosis of the transplanted renal function can be predicted depending on the presence or absence of TLT.

In addition, other specific examples of the test subject in the testing method of the present disclosure include patients with hepatitis C and persons in need of testing the presence or absence of hepatitis C infection. As the symptom of hepatitis C progresses, the TLT tends to be formed in the liver and the degree of maturation thereof tends to increase, and therefore, in the testing method of the present disclosure, in a case where a subject is a person suffering from hepatitis C or a person in need of testing the presence or absence of hepatitis C infection, the presence or absence of hepatitis C infection and the degree of the symptom of hepatitis C can be estimated in addition to the presence or absence of TLT.

Another specific example of the test subject in the testing method of the present disclosure includes a cancer patient. The type of cancer is not particularly limited, and examples thereof include solid cancers such as kidney cancer, gastric cancer, lung cancer, breast cancer, liver cancer, tongue cancer, thyroid cancer, uterine cancer, ovarian cancer, and prostate cancer. When a TLT is formed in a cancer tissue and the degree of maturation thereof is increased, a cancer immune response is improved, and there is a tendency that an improvement in the therapeutic effect of cancer and a good prognosis are recognized, and therefore, in the testing method of the present disclosure, in a case where a cancer patient, particularly a cancer patient to which an immune checkpoint inhibitor is administered, is a subject, the therapeutic effect and prognosis of cancer can be predicted.

### [Body Fluid Sample]

The "body fluid sample" refers to a body fluid such as urine, saliva, blood, sputum, or sweat collected from the subject or a sample prepared from the body fluid. Among such body fluid samples, urine is suitable because the presence or absence of TLT can be tested with high accuracy and non-invasive collection is possible. In addition, the body fluid sample may be subjected to concentration, filtration, and the like as necessary.

### [Measurement of Glutathione Concentration]

In the testing method of the present disclosure, the glutathione concentration in the body fluid sample is measured in order to use the glutathione concentration in the body fluid sample as a biomarker of the TLT.

The glutathione to be measured may be any of reduced glutathione, oxidized glutathione, and total glutathione (both reduced glutathione and oxidized glutathione).

The measurement of the glutathione concentration in the body fluid sample can be performed by a known method. For example, since a kit capable of quantifying the glutathione concentration is commercially available, the glutathione concentration in the body fluid sample can be measured using the commercially available kit. In addition, the measurement of the glutathione concentration in the body fluid sample may be performed using an ion chromatography-Fourier transform mass spectrometer (IC-FTMS), a liquid chromatography-tandem mass spectrometer (LC-MS/MS), or the like.

In addition, when a urine sample is used as a body fluid sample, since the urinary glutathione concentration can vary depending on the urine amount, it is desirable to correct the urinary glutathione concentration by the urinary creatinine concentration or the like. For example, in an embodiment when a urine sample is used as a body fluid sample in the testing method of the present disclosure, a value obtained by dividing a urinary glutathione concentration by a urinary creatinine concentration (creatinine correction value) is used as an index of the urinary glutathione concentration.

### [Determination of Presence or Absence of TLT]

The testing method of the present disclosure can be applied to a testing for determining the presence or absence of a TLT at any lesion site, and a preferred example thereof is an application to a testing for determining the presence or absence of a TLT in the kidney.

There is a correlation between the presence or absence of the TLT and the glutathione concentration in the body fluid sample, and the glutathione concentration in the body fluid sample is low when the TLT is not formed in vivo, and the glutathione concentration in the body fluid sample is high when the TLT is formed in vivo. Therefore, in the testing method of the present disclosure, it is determined that the lower the glutathione concentration in the body fluid sample, the higher the possibility that the TLT is not formed in vivo, and the higher the glutathione concentration in the body fluid sample, the higher the possibility that the TLT is formed in vivo.

According to the testing method of the present disclosure, the determination of the presence or absence of the TLT based on the glutathione concentration in the body fluid sample can be made by comparing with a reference value obtained in advance from a person who knows the presence or absence of the TLT. As used herein, the term "reference value" refers to a value serving as a reference for determination of the presence or absence of the TLT, and specifically, an average value of the glutathione concentration in the body fluid sample obtained from a person who knows the presence or absence of the TLT, a cut-off value obtained from the glutathione concentration in the body fluid sample, and the like.

For example, the average value of the glutathione concentration in the body fluid sample derived from a person in which the TLT is not formed in vivo is obtained in advance, which is used as a reference value, and when the glutathione concentration in the body fluid sample of the subject is equal to or less than the reference value, it can be determined that there is a possibility that the subject does not form the TLT in vivo. For example, the average value of the glutathione concentration in the body fluid sample derived from a person in which the TLT is formed in vivo is obtained in advance, which is used as a reference value, and when the glutathione concentration in the body fluid sample of the subject is equal to or more than the reference value, it can be determined that there is a possibility that the subject forms the TLT in vivo. In addition, for example, the average value of the glutathione concentration in the body fluid sample derived from a person in which the TLT is not formed in vivo and the average value of the glutathione concentration in the body fluid sample derived from a person in which the TLT is formed in vivo are obtained in advance, a cut-off value for dividing the presence or absence of the TLT from these glutathione concentrations is determined in advance, and the cut-off value is used as a reference value, and when the glutathione concentration in the body fluid sample of the subject is equal to or more than the cut-off value, it can be determined that there is a possibility that the subject forms the TLT in vivo.

The reference value is desirably obtained for each type of disease to be tested. For example, if the subject is a kidney disease patient or a person in need of testing the presence or absence of kidney disease infection, an average value of the glutathione concentration in a body fluid sample of a kidney disease patient in which it is known that TLT is not formed in the kidney, a glutathione concentration in a blood fluid sample of a kidney disease patient in which it is known that TLT is formed in the kidney, and/or a cut-off value determined from these glutathione concentrations may be used as a reference value. In addition, for example, when the test subject is a hepatitis C patient or a person in need of testing for presence or absence of hepatitis C infection, an average value of glutathione concentration in a body fluid sample of a hepatitis C patient in which it is known that TLT is not formed in the liver, an average value of glutathione concentration in a body fluid sample of a hepatitis C patient in which it is known that TLT is formed in the liver, and/or a cut-off value determined from these glutathione concentrations may be used as a reference value.

In addition, as shown in the column of Examples, as a result of grouping and analyzing according to the presence or absence of formation of TLT in the kidneys in 47 patients with IgA nephropathy, the average value of the relative values of the urinary glutathione concentration (relative value of the urinary glutathione concentration when the urinary creatinine concentration is 1) of a patient group in which TLT is formed in the kidneys was 0.000740; the average value of relative values of the urinary glutathione concentration of a patient group in which the TLT is not formed in the kidney was 0.000169; and it has been confirmed that the cut-off value of the relative value of the urinary glutathione concentration with which both groups can be distinguished by the sensitivity of 0.7143 and the 1-specificity of 0.00556 is 0.0001647. Therefore, in the testing method of the present disclosure, when a urine sample is used as a body fluid sample and applied to the testing for the presence or absence of TLT in the kidney, the average value or cut-off value of the urinary glutathione concentration can also be used as a reference value.

Based on the presence or absence of the TLT in vivo predicted by the testing method of the present disclosure, it is possible to estimate the presence or absence of disease infection in which the formation of the TLT occurs at the lesion or the degree of symptom of the disease.

For example, in the testing method of the present disclosure, when the subject is a person suffering from a disease accompanied by formation of a TLT other than cancer and it is predicted that there is a possibility that the TLT is formed in vivo, it is presumed that the disease has progressed. Specifically, in the testing method of the present disclosure, when the subject is a kidney disease patient and it is predicted that there is a possibility that the TLT is formed in vivo, it is presumed that kidney disease has progressed.

In addition, there is a possibility that a therapeutic agent targeting TLT is effective for a disease accompanied by formation of TLT (other than a disease accompanied by cancer and infection), and development of a therapeutic agent targeting TLT is expected in the future. Therefore, the testing method of the present disclosure can also be used for determining whether to administer a therapeutic agent targeting a TLT that can be developed in the future. Specifically, in the testing method of the present disclosure, when the subject is a patient suffering from a disease accompanied by formation of a TLT other than that accompanied by cancer and infection and it is predicted that there is a possibility that the TLT is formed in vivo, it can be determined that the subject is suitable for administration of a therapeutic agent targeting the TLT to the patient. In the present disclosure, the "therapeutic agent targeting the TLT" is an agent that stops or suppresses the progression of the TLT or causes the disappearance or decrease of the TLT.

In addition, in the testing method of the present disclosure, when it is predicted that there is a possibility that the TLT is formed even if the subject is a person whose future renal function needs to be predicted and the renal function is normal at the time of the test, it is estimated that there is a possibility that the kidney function may be deteriorated in the future. For example, even if the subject is a person who has undergone kidney transplantation and the function of the transplanted kidney is normal at the time of examination, when it is predicted that there is a possibility that the TLT is formed, it is estimated that there is a possibility that the prognosis of the transplanted renal function will deteriorate.

In addition, in the testing method of the present disclosure, when the subject is a cancer patient and it is predicted that there is a possibility that the TLT is formed in vivo, it is presumed that the cancer immune response is improved.

### 3. TLT Test Kit

Another embodiment of the present disclosure provides a test kit for testing the presence or absence of TLT in vivo, the test kit including a reagent for measuring a glutathione concentration. The test kit of the present disclosure is a test kit used for carrying out the testing method, and the content described in the section "2. Testing Method for TLT" is also incorporated in the test kit of the present disclosure. The test kit of the present disclosure can also be provided as an in-vitro diagnostic drug for testing the presence or absence of TLT in vivo.

The test kit of the present disclosure only needs to include a reagent for measuring the glutathione concentration, and may contain, for example, an enzyme, a coenzyme, a buffer, a substrate, an internal standard substance, and the like. Examples of the reagent for measuring the glutathione concentration include a combination of glutathione reductase, NADPH, and 5-5'-dithiobis(2-nitrobenzoic acid) (DTNB). Another example of the reagent for measuring the glutathione concentration includes a combination of glutathione S-transferase, luciferin-NT (Promega), and luciferase.

When the glutathione concentration in the urine sample is measured using the test kit of the present disclosure, the test kit of the present disclosure may include a reagent for measuring the creatinine concentration in order to correct the glutathione concentration in the urine sample. The reagent for measuring the creatinine concentration only needs to include, for example, an enzyme for detecting creatinine by an enzymatic measurement method, and specific examples thereof include creatininase, creatinase, sarcosine oxidase, and a detection reagent of active oxygen (for example, peroxidase, 4-aminoantipyrine, and N-ethyl-N-(2-hydroxy-3-sulfopropyl)-m-toluidine). Examples

The present disclosure is not intended to be limited in any way to the embodiments of the invention described above and the description of Examples below. Various modifications that can be easily conceived by those skilled in the art without departing from the scope of the claims are also included in the present invention. The contents of the documents and the like shown in the present specification are cited by incorporating the entire contents thereof.

### 1. Analysis Using Renal TLT Model

### 1-1. Test Materials and Methods

### (1) Mice

From Japan SCL Inc., 12 month-old C57BL6J mice were purchased. All the mice were kept in a specific pathogen-free (SPF) environment in Kyoto University animal experiment facility. All animal experiments were performed in accordance with the Guide for the Care and Use of Laboratory Animals of the National Institutes of Health (NIH) with the approval of the Institutional Animal Care and Use Committee, Kyoto University.

### (2) Kidney TLT Model

Mice were subjected to skin incision under general anesthesia with 2% isoflurane, and the left kidney was exposed to the body surface. Ischemia reperfusion injury (IRI) was induced by fully occluding the renal arteries on both sides for 45 minutes using a vascular clamp followed by opening and closing. Urine was collected every week from the day of IRI induction. The mice were sacrificed 45 days after the day of IRI induction, and renal tissues were collected (n = 5). In addition, urine and renal tissues were collected under the same conditions as described above except that IRI induction was not performed (control) (n = 5).

### (3) Renal Tissue Staining (HE Staining and Immunostaining)

Hematoxylin and eosin (HE) staining of renal tissue was performed in the following procedure. Immediately after the sacrifice, the renal tissue instantaneously frozen with dry ice was sliced to 8 µm with cryostat (CM3050, Leica) as unembedded. The sliced tissue was fixed to a slide glass (Matsunami Glass Ind.) and allowed to permeate a hematoxylin stain solution for 4 minutes. Thereafter, the sample was washed with running water for 15 minutes and was allowed to be permeated with an eosin stain for 2 minutes, and subjected to HE staining.

Immunostaining of renal tissue was performed by the following procedure. First, renal tissue sections were blocked for 1 hour at room temperature using phosphate-buffered saline (PBS) containing 5% normal goat serum. Next, an anti-B220 antibody (557390, BD Bioscience) and an anti-CD3e antibody (55027, BD Bioscience) were reacted overnight at 4°C as primary antibodies. Subsequently, the appropriate secondary antibodies were reacted for 1 hour at room temperature and counterstained with 4',6-diamidino-2-phenylindole (DAPI). Observation was performed with a confocal microscope (FV1000D; Olympus Corporation).

### (4) Renal Tissue Mass Imaging

Immediately after the sacrifice, the kidney tissue instantaneously frozen with dry ice was sliced to 8 µm with cryostat (CM3050, Leica) as unembedded. The sliced tissue was fixed on indium tin oxide (ITO) coated glass slides (Brucker Daltonics). Matrix-assisted laser desorption ionization mass spectrometry (MALDI-IMS) was performed using 9-aminoacridine (10 mg/mL, dissolved in 80% ethanol) as a matrix. MALDI imaging was performed using a matrix-assisted laser desorption ionization time of flight (MALDI-TOF) mass spectrometer (UltraFlextreme, Brucker Daltonics) equipped with a Nd: YAG laser. A raster scan was performed every 50 mm, and data was collected in a negative reflection mode. Image reconstruction was performed using FlexImaging 4.1 software (Brucker Daltonics).

### (5) Renal Tissue Metabolome Analysis

The tissue section embedded in SCEM (Super Cryoembedding Medium) was dissolved in 500 µL of methanol using a homogenizer (Finger Masher, AM79330, Sarstedt, Tokyo, Japan). Thereafter, an equal amount of chloroform and 0.4 times the amount of ultrapure water were added and mixed. The mixture was centrifuged at 15,000 G and 4°C for 15 minutes. The obtained supernatant was filtered using an ultra filter tube (Ultrafree-MC, UFC3 LCC NB; Human Metabolome Technologies, Tsuruoka, Japan). The filtrate was concentrated using a centrifugal concentrator (SpeedVac; Thermo, Yokohama, Japan). The obtained filtrate after concentration was dissolved in 50 µL of ultrapure water and subjected to metabolome analysis using a mass spectrometer. The metabolome analysis was performed by the following method while being divided into anionic metabolites and cationic metabolites. For the analysis of the data, the results of cationic metabolites for which more precise values were required were used.

For metabolome analysis of anionic metabolites, an ion chromatograph (IC) - Fourier transform mass spectrometer (FTMS) was used. Specifically, anionic metabolites were measured with an orbitrap mass spectrometer (Q-Exactive focus, Thermo Fisher Scientific) connected to a high-performance IC system (ICS-5000+, Thermo Fisher Scientific), and metabolite quantification was performed with high selectivity and high sensitivity by IC separation and Fourier transform mass spectrometry principle. The ion chromatograph was equipped with an anion electrolysis suppressor (Thermo Scientific Dionex AERS 500), and the potassium hydroxide gradient was converted into pure water before entering the mass spectrometer. Separation was performed using a Dionex (trademark) IonPac (trademark) AS11-HC-4µm IC column (Thermo Scientific). The IC flow rate was 0.25 mL/min and the post column was supplemented with methanol makeup flow at 0.18 mL/min. The concentration gradient conditions of potassium hydroxide for IC separation are as follows. The column temperature was 30°C at 1 mM to 100 mM (0 to 40 minutes), 100 mM (40 to 50 minutes), and 1 mM (50.1 to 60 minutes). The orbitrap mass spectrometer was operated in ESI negative mode for all detections. Full mass scan (m/z: 70 to 900) was performed at a resolution of 70,000. The automatic gain control (AGC) target was set to 3 x 106 ions and the maximum ion injection time was set to 100 ms. The ionization parameters of the ion source were set at a spray voltage of 3 kV, a transfer temperature of 320°C, an S-Lens level of 50, a heater temperature of 300°C, a sheath gas flow rate of 36, and aux gas flow rate of 10.

For metabolome analysis of cationic metabolites, liquid chromatography-tandem mass spectrometry (LC-MS/MS) was used. Specifically, a triple quadrupole mass spectrometer (LCMS-8060, SHIMADZU) equipped with an orthogonal electrospray (ESI) ion source was used in positive and negative ESI, multiple reaction monitoring (MRM) mode. The samples were separated on a Discovery HS F5-3 column (2.1 mm ID x 150 mm L, 3-µm particle, Sigma-Aldrich) using a step gradient of mobile phase A (0.1% formic acid) and mobile phase B (0.1% acetonitrile). A step gradient was performed under the conditions of a ratio of mobile phase A:mobile phase B of 100:0 (0 to 5 minutes), 75:25 (5 to 11 minutes), 65:35 (11 to 15 minutes), 5:95 (15 to 20 minutes), and 100:0 (20 to 25 minutes), a flow rate of 0.25 mL/min, and a column temperature of 40°C.

### (6) Urine Metabolome Analysis

After 50 µL of frozen urine was mixed with 500 µL of methanol and a standard substance (2-morpholinoethanesulfonic acid), equal amounts of chloroform and 0.4 volumes of ultrapure water (LC/MS grade, FUJIFILM Wako) were added thereto. The resulting suspension was centrifuged at 2,800 g at 4°C for 15 minutes. After centrifugation, the aqueous phase was subjected to ultrafiltration using an ultrafiltration tube (Ultrafree MC-PLHCC; Human Metabolome Technologies). The filtrate was concentrated using a vacuum concentrator (SpeedVac, Thermo Fisher Scientific). The concentrated filtrate was dissolved in 50 µL of ultrapure water, and subjected to analysis by IC-FTMS and LC-MS/MS. In order to correct the loss of endogenous metabolites during sample preparation, the urinary metabolite concentration was calculated as a relative value corrected by urinary creatinine concentration (relative value with creatinine concentration as 1). Analysis by IC-FTMS and LC-MS/MS was performed in the same manner as in the case of renal tissue metabolome analysis.

### 1-2. Results

HE staining of the renal tissue of a mouse in which ischemia reperfusion injury was induced showed that a tissue presumed to be TLT was observed in the renal tissue (Fig. 2b). In addition, CD3e as a T cell marker and B220 as a B cell marker were localized in the tissue presumed to be TLT by HE staining (Fig. 2b), and it was confirmed to be TLT composed of an aggregate of T cells and B cells.

In addition, from the result of mass imaging of the renal tissue of a mouse in which ischemia reperfusion injury was induced, it was confirmed that glutathione (oxidized form and reduced form) was accumulated in the TLT (Figs. 2c and 2d). Furthermore, in the mouse in which ischemia reperfusion injury was induced, the amount of glutathione (oxidized and reduced forms) extracted from the kidney and its decomposition product (dipeptide composed of Cys-Gly) was significantly increased as compared with the control mouse (Fig. 2e).

Furthermore, in the mice in which ischemia reperfusion injury was induced, the urinary reduced glutathione concentration was significantly increased as compared with the control mice (Fig. 3).

From the above results, it became clear that glutathione was highly accumulated in the TLT formed in the kidney, and an increase in the urinary glutathione concentration was observed when the TLT was formed.

### 2. Analysis Using Urine Sample of Patient with IgA Nephropathy

### 2-1. Test Materials and Methods

### (1) Patient

There were 46 patients with IgA nephropathy diagnosed by renal biopsy between 2014 and 2018 at Kyoto University Hospital who were retrospectively screened. From 46 cases, 46 cases in which kidney biopsy tissues and urine specimens (frozen urine) remained and consent on this test was obtained were analyzed. Note that patients who received immunosuppressive therapy within 1 year before renal biopsy, patients with systemic lupus erythematosus (SLE), patients with rheumatoid arthritis, patients with purpura nephritis, and patients with liver cirrhosis are excluded from the analysis targets.

### (2) Identification of TLT

In this study, an aggregate of organized lymphocytes with signs of proliferation was defined as TLT, similar to the criteria presented in NPL 4. Since the size of TLT varied, an aggregate of 61 or more lymphocytes (T cells and B cells) and containing at least 1 Ki67 positive cell was defined as TLT in this test. Quantification of the number of TLTs and determination of the stage were performed in a blinded manner according to the method described in NPL 5. Briefly, first, mononuclear infiltration in the renal stroma was confirmed in PAS-stained sections. Next, the TLT was diagnosed by evaluating mononuclear infiltration by performing immunofluorescence staining of 1) CD3ε and CD20, and 2) Ki67 and CD21 in two serial sections. After diagnosis of TLT, the stages of TLT were classified according to the following criteria: i) TLT without follicular dendritic cells (FDC) or germinal centers was defined as stage I, ii) TLT with FDC and lacking germinal centers was defined as stage II, and iii) TLT with FDC and germinal centers was defined as stage III. FDC was defined as cells strongly expressing CD21 within the TLT. Germinal center was defined as microstructures containing clusters of 16 or more Ki67 positive cells within the B cell area.

Of the 46 cases of IgA nephropathy patients to be analyzed, 18 cases had TLT, and 28 cases had no TLT. When the stage of TLT was determined for 18 cases in which TLT was observed, 14 cases were determined as stage I, 4 cases were determined as stage II, and 0 cases were determined as stage III.

### (3) Urine Metabolome Analysis

The urinary reduced glutathione concentration was measured in the same manner as described in "1. Analysis using renal TLT model" above.

### (4) Logistic Regression Analysis and ROC Analysis

Logistic regression analysis and ROC analysis were performed using JMP Pro software (version 16). Logistic regression was performed using oxidized glutathione as an explanatory variable to predict the presence or absence of TLT. Using the ROC curve and the Youden index, the cut-off value of the urinary oxidized glutathione concentration for predicting the presence of TLT was determined, and a value equal to or more than the cut-off value was defined as TLT positive.

### 2-2. Results

When the urinary reduced glutathione concentration was measured by dividing the IgA nephropathy patients into the group with TLT and the group without TLT, the average value of the relative value of the urinary oxidized glutathione concentration of the patient group having TLT was 0.000740, and the average value of the relative value of the urinary oxidized glutathione concentration of the patient group not having TLT was 0.000169, and in the patient group having TLT, the urinary reduced glutathione concentration significantly increased as compared with the patient group not having TLT (Fig. 4).

In addition, patients in the group with TLT were positive (1) and patients in the group without TLT were negative (0), and univariate analysis by logistic regression was performed using the relative value of the urinary reduced glutathione concentration as an explanatory variable. As a result, the AUC (Area Under Curve) was 0.893, and the relative value of the urinary reduced glutathione concentration at which the sensitivity - (1- specificity) was maximum was 0.0001647 (Fig. 5). When the relative value was used as a cut-off value, the sensitivity was 0.7143, and the 1-specificity was 0.0556 (Fig. 5).

From the above results, it has become clear that the urinary glutathione concentration significantly increases in IgA nephropathy patients having TLT, and the presence or absence of TLT can be predicted by using the urinary glutathione concentration as an index.

## Claims

1. A testing method for determining presence or absence of a tertiary lymphoid tissue in a subject, the testing method comprising a step of measuring a glutathione concentration in a body fluid sample collected from the subject.

2. The testing method according to claim 1, wherein the body fluid sample is a urine sample.

3. The testing method according to claim 1 or 2, wherein the glutathione concentration is a reduced glutathione concentration.

4. The testing method according to claim 2, further comprising a step of measuring a creatinine concentration in the urine sample.

5. The testing method according to claim 1 or 2, wherein the subject is a kidney disease patient, a person in need of testing for presence or absence of kidney disease infection, or a person who has undergone kidney transplantation, the testing method being performed for testing presence or absence of a tertiary lymphoid tissue in a kidney.

6. A test kit for testing presence or absence of a tertiary lymphoid tissue in vivo, the test kit comprising a reagent for measuring a glutathione concentration.

7. Use of a reagent for measuring a glutathione concentration in manufacture of a test kit for testing presence or absence of a tertiary lymphoid tissue in a body.
